(19) Europäisches Patentamt
European Patent Office
Office européen des brevets

(11) Veröffentlichungsnummer: **0 043 436**
**B1**

(12) # EUROPÄISCHE PATENTSCHRIFT

(45) Veröffentlichungstag der Patentschrift: **31.08.83**

(51) Int. Cl.³: **A 61 K 7/13**

(21) Anmeldenummer: **81104079.9**

(22) Anmeldetag: **27.05.81**

(54) **Mittel und Verfahren zum Färben von Haaren.**

(30) Priorität: **07.07.80 DE 3025715**

(43) Veröffentlichungstag der Anmeldung: **13.01.82 Patentblatt 82/2**

(45) Bekanntmachung des Hinweises auf die Patenterteilung: **31.08.83 Patentblatt 83/35**

(84) Benannte Vertragsstaaten: **DE FR GB IT SE**

(56) Entgegenhaltungen:
**BE-A-660 519**
**DE-A-2 840 830**
**FR-A-2 132 214**

**BEILSTEINS HANDBUCH DER ORGANISCHEN CHEMIE, 4. Auflage, Hauptwerk, Band 6, 1923, Springer Verlag Berlin, DE**

**BEILSTEINS HANDBUCH DER ORGANISCHEN CHEMIE, 4. Auflage, 1. Ergänzungswerk, Band 6, 1931, Springer Verlag Berlin, DE**

**BEILSTEINS HANDBUCH DER ORGANISCHEN CHEMIE, 4. Auflage, 4. Ergänzungswerk, Band 6, 1980, Springer Verlag Berlin, DE**

(73) Patentinhaber: **Wella Aktiengesellschaft, Berliner Allee 65, D-6100 Darmstadt (DE)**

(72) Erfinder: **Konrad, Eugen, Mecklenburger Strasse 101, D-6100 Darmstadt (DE)**
Erfinder: **Braun, Hans-Jürgen, Dr., Impasse de la Colline 3, CH-1723 Marly (CH)**
Erfinder: **Mager, Herbert, Dr., Beaumont 5, CH-1700 Fribourg (CH)**

**EP 0 043 436 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Mittel und Verfahren zum Färben von Haaren

Die Erfindung betrifft ein Mittel und ein Verfahren zum oxidativen Färben von Haaren auf der Basis eines substituierten Phenols der allgemeinen Formel

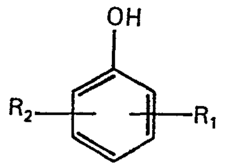

wobei $R_1$ einen Hydroxyalkylrest mit 1 bis 4 Kohlenstoffatomen bedeutet und $R_2$ ein Wasserstoffatom oder einen Alkylrest mit 1 bis 4 Kohlenstoffatomen darstellt, als Kupplersubstanz.

Für die Haarfärbung haben Oxidationsfarbstoffe eine wesentliche Bedeutung erlangt. Die Färbung entsteht hierbei durch die Reaktion bestimmter Entwicklersubstanzen mit bestimmten Kupplersubstanzen in Gegenwart eines geeigneten Oxidationsmittels.

Als Entwicklersubstanzen werden hauptsächlich 1,4-Diaminobenzol, 2,5-Diaminotoluol, 3-Methyl-4-aminophenol und p-Aminophenol eingesetzt. Die bevorzugt verwendeten Kupplersubstanzen sind α-Naphthol, Resorcin, 4-Chlorresorcin, 2-Methylresorcin, m-Aminophenol, 5-Amino-o-kresol und Derivate des m-Phenylendiamins wie m-Toluylendiamin und 2,4-Diaminoanisol. Diese Derivate sowie das m-Phenylendiamin selbst haben hierbei wegen ihrer Fähigkeit, bei der oxidativen Kupplung mit 1,4-Diaminobenzol bzw. 1,4-Diaminobenzolderivaten Blautöne zu erzeugen, als sogenannte Blaukuppler Bedeutung erlangt.

An Oxidationsfarbstoffe, die zum Färben von menschlichen Haaren Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen. Weiterhin ist es erforderlich, dass durch Kombination geeigneter Entwickler- und Kupplerkomponenten eine breite Palette unterschiedlicher Farbnuancen erzeugt werden kann. Ausserdem wird für die erzielbaren Haarfärbungen eine gute Licht-, Dauerwell-, Säure- und Reibechtheit gefordert. Auf jeden Fall aber müssen solche Haarfärbungen ohne Einwirkung von Licht, chemischen Mitteln und Reibung über einen Zeitraum von mindestens 4 bis 6 Wochen stabil bleiben.

Das zurzeit in Haarfärbemitteln als Blaukuppler verwendete m-Phenylendiamin, dessen Derivate m-Toluylendiamin und 2,4-Diaminoanisol sowie auch in neuerer Zeit empfohlene Blaukuppler, wie beispielsweise 1-Hydroxy-3-amino-6-chlorbenzol und 2,4-Diaminophenoxyethanol, können die vorstehend genannten Anforderungen jedoch noch nicht zufriedenstellend erfüllen.

Es bestand daher die Aufgabe, nach einem Haarfärbemittel sowie einem Haarfärbeverfahren zu suchen, bei dem die gestellten Anforderungen in optimaler Weise erfüllt werden.

Hierzu wurde nun gefunden, dass Mittel zum oxidativen Färben von Haaren, welche eine Kombination von in der Haarfärbung bekannten Entwicklersubstanzen mit mindestens einem substituierten Phenol der allgemeinen Formel

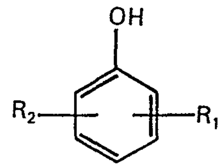

wobei $R_1$ einen Hydroxyalkylrest mit 1 bis 4 Kohlenstoffatomen bedeutet und $R_2$ ein Wasserstoffatom oder einen Alkylrest mit 1 bis 4 Kohlenstoffatomen darstellt, auch in Form des Phenolates mit organischen oder anorganischen Basen, als Kupplersubstanz enthalten, in hervorragendem Masse der gestellten Aufgabe gerecht werden.

Die als Kupplersubstanzen in den erfindungsgemässen Haarfärbemitteln enthaltenen substituierten Phenole der angegebenen Formel sind unter Zusatz von Alkalien wie beispielsweise Natriumhydroxid gut in Wasser löslich und weisen ausserdem eine unerwartet gute Lagerbeständigkeit, insbesondere als Bestandteil der erfindungsgemässen Haarfärbemittel, auf.

Beispiele für geeignete, in den erfindungsgemässen Haarfärbemitteln enthaltene Kupplersubstanzen der angegebenen Formel sind insbesondere

3-(β-Hydroxyethyl)-phenol
3-(γ-Hydroxypropyl)-phenol
3-(β-Hydroxyethyl)-6-methyl-phenol
2-(β-Hydroxyethyl)-5-methyl-phenol
2-Hydroxymethyl-5-methyl-phenol
2-Hydroxymethyl-6-methyl-phenol

In den Haarfärbemitteln sollen die genannten Kupplersubstanzen, von denen 2-Hydroxymethyl-5-methyl-phenol und 3-(β-Hydroxyethyl)-6-methyl-phenol bevorzugt werden, in einer Konzentration von 0,01 bis 3,0 Gewichtsprozent, insbesondere 0,1 bis 2,0 Gewichtsprozent, enthalten sein.

Ausserdem können die Haarfärbemittel zusätzlich bekannte Kupplersubstanzen, insbesondere α-Naphthol, 3,4-Diaminobenzoesäure, Resorcin, 4-Chlorresorcin, 2-Methylresorcin, m-Aminophenol und 3-Amino-6-methyl-phenol enthalten. Ferner können auch 4-Hydroxy-1,2-methylendioxybenzol und 4-Amino-1,2-methylendioxybenzol vorteilhaft als Kupplersubstanz eingesetzt werden.

Von den bekannten Entwicklersubstanzen kommen als Bestandteil der erfindungsgemässen Haarfärbemittel vor allem 1,4-Diaminobenzol, 2,5-Diaminotoluol, 2,5-Diaminoanisol, p-Aminophenol sowie 3-Methyl-4-aminophenol in Betracht. Auch 2,5-Diaminobenzylalkohol und 2-(β-Hydroxyethyl)-1,4-diaminobenzol sind vorteilhaft als Entwicklersubstanz verwendbar.

Sowohl die erfindungsgemäss verwendeten substituierten Phenole der angegebenen Formel als auch die bekannten Kuppler- und Entwicklersubstanzen können in den Haarfärbemitteln jeweils allein oder im Gemisch miteinander enthalten sein.

Die Gesamtmenge der in den hier beschriebenen Haarfärbemitteln enthaltenen Entwicklersubstanz-Kupplersubstanz-Kombination soll 0,1 bis 5,0 Gewichtsprozent, insbesondere 0,5 bis 3,0 Gewichtsprozent, betragen.

Die Entwicklerkomponenten werden im allgemeinen in etwa äquimolaren Mengen, bezogen auf die Kupplerkomponenten, eingesetzt. Es ist aber nicht nachteilig, wenn die Entwicklerkomponente diesbezüglich in einem gewissen Überschuss oder Unterschuss vorhanden ist.

Weiterhin können die Haarfärbemittel dieser Anmeldung zusätzlich andere Farbkomponenten, beispielsweise 6-Amino-3-methyl-phenol, 6-Amino-2-methyl-phenol und 6-Amino-3-ethoxy-phenol, sowie ferner übliche direktziehende Farbstoffe, zum Beispiel Triphenylmethanfarbstoffe wie Diamond Fuchsin (C.I. 42 510) und Leather Ruby HF (C.I. 42 520), aromatische Nitrofarbstoffe wie 2-Nitro-1,4-diaminobenzol und 2-Amino-4-nitrophenol, Azofarbstoffe wie Acid Brown 4 (C.I. 14 805) und Acid Blue 135 (C.I. 13 385), Anthrachinonfarbstoffe wie Disperse Red 15 (C.I. 60 710) und Disperse Violet 1 (C.I. 61 100), ausserdem 1,4,5,8-Tetraamino-anthrachinon und 1,4-Diamino-anthrachinon enthalten.

Darüber hinaus können in den Haarfärbemitteln noch weitere übliche kosmetische Zusätze, beispielsweise Antioxidantien wie Ascorbinsäure oder Natriumsulfit, Parfümöle, Komplexbildner, Netzmittel, Emulgatoren, Verdicker, Pflegestoffe und andere vorhanden sein.

Die Zubereitungsform kann die einer Lösung, vorzugsweise einer Creme, eines Gels oder einer Emulsion sein. Ihre Zusammensetzung stellt eine Mischung der Farbstoffkomponenten mit den für solche Zubereitungen üblichen Bestandteilen dar. Als übliche Bestandteile von Cremes, Emulsionen oder Gelen kommen beispielsweise Netzmittel oder Emulgatoren aus den Klassen der anionischen, kationischen oder nichtionogenen oberflächenaktiven Substanzen wie Fettalkoholsulfate, Alkylsulfonate, Alkylbenzolsulfonate, Alkyltrimethylammoniumsalze, oxethylierte Fettalkohole, oxethylierte Nonylphenole, Fettsäurealkanolamide, ferner Verdicker wie höhere Fettalkohole, Stärke, Cellulosederivate, Paraffinöl und Fettsäuren sowie ausserdem Pflegestoffe wie Lanolinderivate, Cholesterin und Pantothensäure in Betracht. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,1 bis 25 Gewichtsprozent in den Zubereitungen enthalten sein können.

Je nach Zusammensetzung können die erfindungsgemässen Haarfärbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können aber auch organische Amine, zum Beispiel Monoethanolamin oder Triethanolamin, Verwendung finden.

Bei dem erfindungsgemässen Verfahren zur oxidativen Färbung von Haaren vermischt man die Haarfärbemittel, welche eine Kombination von in der Haarfärbung bekannten Entwicklersubstanzen mit mindestens einem substituierten Phenol der angegebenen Formel als Kupplersubstanz sowie gegebenenfalls zusätzlich bekannte Kupplersubstanzen enthalten, kurz vor dem Gebrauch mit einem Oxidationsmittel und trägt dieses Gemisch auf das Haar auf. Als Oxidationsmittel zur Entwicklung der Haarfärbung kommt hauptsächlich Hydrogenperoxid, beispielsweise als 6%ige wässrige Lösung bzw. dessen Additionsverbindungen an Harnstoff, Melamin oder Natriumborat in Betracht. Man lässt das Gemisch bei 15 bis 50°C etwa 10 bis 45 Minuten lang, vorzugsweise 30 Minuten lang, auf das Haar einwirken, spült sodann das Haar mit Wasser aus und trocknet. Gegebenenfalls wird im Anschluss an diese Spülung mit einem Shampoo gewaschen und eventuell mit einer schwachen organischen Säure, wie zum Beispiel Zitronensäure oder Weinsäure, nachgespült.

Die in den erfindungsgemässen Mitteln enthaltenen substituierten Phenole gemäss der Formel sind bekannte Verbindungen oder nach üblichen in der Literatur beschriebenen organischen Syntheseverfahren darstellbare Verbindungen.

Beispielsweise geht man bei der Herstellung des 2-Hydroxymethyl-5-methyl-phenols von der in technischen Mengen verfügbaren m-Kresotinsäure aus, führt diese durch Veresterung[1] in den entsprechenden m-Kresotinsäureester über und reduziert[2] mit Lithiumaluminiumhydrid. Auf die gleiche Weise kann 2-Hydroxymethyl-6-methylphenol aus o-Kresotinsäure hergestellt werden.

Zur Synthese von 3-(β-Hydroxyethyl)-6-methyl-phenol wird 4-Methyl-phenylessigsäure durch Nitrierung zu 4-Methyl-3-nitro-phenylessigsäure umgesetzt. Anschliessend erfolgt die selektive Reduktion[3] der Carboxyl-Gruppe mit Borhydrid, wobei 2-(4′-Methyl-3′-nitro-phenyl)-ethanol erhalten wird. Hierauf wird die Nitro-Gruppe mit Wasserstoff in Gegenwart eines Patinkatalysators zur Amino-Gruppe reduziert. Schliesslich wird die Amino-Gruppe bei der Umsetzung mit salpetriger Säure unter Stickstoffeliminierung durch eine OH-Gruppe ersetzt.

---

[1] Houben-Weyl, Methoden der Organischen Chemie, 4. Auflage, VIII, 525.

[2] Organikum, VEB Deutscher Verlag der Wissenschaften, Berlin (1976) 614.
Organic Reactions *6* (1951) 469.

[3] H.C. Brown, B.C. Subba Rao, J. Am. Chem. Soc. *82* (1960) 681.

Hinsichtlich der färberischen Möglichkeiten bieten die erfindungsgemässen Haarfärbemittel je nach Art und Zusammensetzung der Farbkomponenten eine breite Palette verschiedener Farbnuancen, welche sich von blonden über braune, aschige, purpurne, violette, goldene bis hin zu blauen sowie matten Farbtönen erstrecken. Hierbei zeichnen sich die Farbtöne durch ihre besondere Beständigkeit und Trageechtheit aus. Darüber hinaus ist das beobachtete Färbeergebnis unabhängig von der Struktur des jeweilig gefärbten Haares. Das bedeutet, dass die mit den erfindungsgemässen Haarfärbemitteln erzielten Farbtöne gut reproduzierbar sind.

Von wesentlicher Bedeutung ist ferner der durch die Verwendung der substituierten Phenole der angegebenen Formel in den hier beschriebenen Haarfärbemitteln in toxikologischer und dermatologischer Hinsicht, zum Beispiel gegenüber den bekannten Blaukupplern 2,4-Diaminotoluol, 2,4-Diaminoanisol und 1,3-Diaminobenzol, erzielte Fortschritt, welcher auf den beiden in den substituierten Phenolen enthaltenen Hydroxylgruppen (für den Fall, dass $R_1$ einen Hydroxyalkylrest darstellt) und der damit verbundenen Abnahme der Lipoidlöslichkeit beruht.

Von besonderer Bedeutung ist weiterhin, dass mit einigen der hier beschriebenen substituierten Phenole gemäss der Formel, zum Beispiel mit 2-Hydroxymethyl-5-methyl-phenol, erstmals neue Blaukuppler vorliegen, die im Gegensatz zu Kupplern ähnlicher Strukturformel – wie zum Beispiel 2,5-Dimethylphenol und m-Kresol – überraschenderweise dem damit gefärbten Haar keinen unangenehmen, stechenden kresolischen Geruch verleihen.

Schliesslich ist mit Hilfe der erfindungsgemässen Haarfärbemittel auch eine Anfärbung von ergrauten, chemisch nicht vorgeschädigtem Haar problemlos und mit sehr guter Deckkraft möglich.

Die nachfolgenden Beispiele sollen den Erfindungsgegenstand näher erläutern.

Beispiel 1 Haarfärbemittel in Gelform

| | |
|---|---|
| 0,40 g | 2-Hydroxymethyl-5-methyl-phenol |
| 0,70 g | 2,5-Diaminotoluolsulfat |
| 0,12 g | Natriumhydroxid, fest |
| 0,30 g | Ascorbinsäure |
| 1,00 g | Hydroxyethylcellulose, hochviskos |
| 5,00 g | Laurylalkohol-diglykolethersulfat, Natriumsalz (28%ige wässrige Lösung) |
| 10,00 g | Ammoniak, 22%ig |
| 82,48 g | Wasser |
| 100,00 g | |

50 g des obigen Haarfärbemittels werden kurz vor dem Gebrauch mit 50 ml Hydrogenperoxidlösung (6%ig) vermischt und das Gemisch anschliessend auf blonde menschliche Haare aufgetragen. Nach einer Einwirkungszeit von 30 Minuten bei etwa 40°C wird mit Wasser gespült und getrocknet. Das Haar ist mit einem intensiven Blauton gefärbt.

Beispiel 2 Haarfärbemittel in Gelform

| | |
|---|---|
| 0,40 g | 2-Hydroxymethyl-5-methyl-phenol |
| 0,80 g | 2,5-Diaminobenzylalkohol-dihydrochlorid |
| 0,12 g | Natriumhydroxid, fest |
| 0,30 g | Ascorbinsäure |
| 15,00 g | Ölsäure |
| 7,00 g | Isopropanol |
| 10,00 g | Ammoniak, 22%ig |
| 66,38 g | Wasser |
| 100,00 g | |

Man vermischt kurz vor dem Gebrauch 50 g dieses Haarfärbemittels mit 50 ml Hydrogenperoxidlösung (6%ig) und lässt das Gemisch 30 Minuten lang bei 40°C auf blonde menschliche Haare einwirken. Danach wird mit Wasser gespült und getrocknet. Das Haar hat eine grauviolett-bläuliche Färbung erhalten.

Beispiel 3 Haarfärbemittel in Cremeform

| | |
|---|---|
| 0,15 g | 2-Hydroxymethyl-5-methyl-phenol |
| 0,80 g | 2,5-Diaminotoluolsulfat |
| 0,20 g | Resorcin |
| 0,04 g | m-Aminophenol |
| 0,05 g | Natriumhydroxid, fest |
| 3,50 g | Laurylalkohol-diglykolethersulfat, Natriumsalz (28%ige wässrige Lösung) |
| 0,30 g | Natriumsulfit, wasserfrei |
| 15,00 g | Cetylalkohol |
| 5,00 g | Ammoniak, 22%ig |
| 74,96 g | Wasser |
| 100,00 g | |

50 g dieses Haarfärbemittels werden kurz vor dem Gebrauch mit 50 ml Hydrogenperoxidlösung (6%ig) vermischt und das Gemisch anschliessend auf blonde menschliche Haare aufgebracht. Nach einer Einwirkungszeit von 30 Minuten bei 40°C wird mit Wasser gespült und getrocknet. Das Haar ist hellaschbraun gefärbt.

Alle in der vorliegenden Anmeldung angegebenen Prozentzahlen stellen Gewichtsprozente dar.

**Patentansprüche**

1. Mittel zum oxidativen Färben von Haaren, dadurch gekennzeichnet, dass es eine Kombination von in der Haarfärbung bekannten Entwicklersubstanzen mit 0,01 bis 3,0 Gewichtsprozent mindestens eines substituierten Phenols der allgemeinen Formel

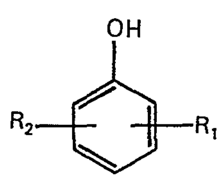

wobei $R_1$ einen Hydroxyalkylrest mit 1 bis 4 Kohlenstoffatomen bedeutet und $R_2$ ein Wasserstoffatom oder einen Alkylrest mit 1 bis 4 Kohlenstoffatomen darstellt, auch in Form des Phenolates

mit organischen oder anorganischen Basen, als Kupplersubstanz sowie gegebenenfalls zusätzlich bekannte Kupplersubstanzen enthält, so dass die Gesamtmenge der enthaltenen Entwicklersubstanz-Kupplersubstanz-Kombination 0,1 bis 5,0 Gewichtsprozent beträgt.

2. Mittel nach Anspruch 1, dadurch gekennzeichnet, das das substituierte Phenol ausgewählt ist aus 3-(β-Hydroxyethyl)-phenol, 3-(γ-Hydroxypropyl)-phenol, 3-(β-Hydroxyethyl)-6-methyl-phenol, 2-(β-Hydroxyethyl)-5-methyl-phenol, 2-Hydroxymethyl-5-methyl-phenol und 2-Hydroxymethyl-6-methyl-phenol.

3. Mittel nach Anspruch 1 und 2, dadurch gekennzeichnet, dass es die erfindungsgemässen Kupplersubstanzen in einer Konzentration von 0,1 bis 2,0 Gewichtsprozent enthält.

4. Mittel nach Anspruch 1 bis 3, dadurch gekennzeichnet, dass es mindestens eine der Entwicklersubstanzen 1,4-Diaminobenzol, 2,5-Diaminotoluol, 2,5-Diaminoanisol, 2-(β-Hydroxyethyl)-1,4-diaminobenzol, 2,5-Diaminobenzylalkohol, p-Aminophenol und 3-Methyl-4-aminophenol enthält.

5. Mittel nach Anspruch 1 bis 4, dadurch gekennzeichnet, dass es mindestens eine der Kupplersubstanzen α-Naphthol, 3,4-Diaminobenzoesäure, Resorcin, 4-Chlorresorcin, 2-Methylresorcin, m-Aminophenol, 3-Amino-6-methylphenol, 4-Hydroxy-1,2-methylendioxybenzol und 4-Amino-1,2-methylendioxybenzol enthält.

6. Mittel nach Anspruch 1 bis 5, dadurch gekennzeichnet, dass die Gesamtmenge der enthaltenen Entwicklersubstanz-Kupplersubstanz-Kombination 0,5 bis 3,0 Gewichtsprozent beträgt.

7. Mittel nach Anspruch 1 bis 6, dadurch gekennzeichnet, dass es zusätzlich als Farbkomponenten 6-Amino-3-methylphenol, 6-Amino-2-methylphenol und 6-Amino-3-ethoxyphenol enthält.

8. Mittel nach Anspruch 1 bis 7, dadurch gekennzeichnet, dass es zusätzlich mindestens einen der direktziehenden Farbstoffe Diamond Fuchsin (C.I. 42 510), Leather Ruby HF (C.I. 42 520), 2-Nitro-1,4-diaminobenzol, 2-Amino-4-nitrophenol, Acid Brown 4 (C.I. 14 805), Acid Blue 135 (C.I. 13 385), Disperse Red 15 (C.I. 60 710), Disperse Violet 1 (C.I. 61 100), 1,4,5,8-Tetraaminoanthrachinon und 1,4-Diaminoanthrachinon enthält.

9. Mittel nach Anspruch 1 bis 8, dadurch gekennzeichnet, dass es zusätzlich Antioxidantien, vorzugsweise Ascorbinsäure oder Natriumsulfit, enthält.

10. Mittel nach Anspruch 1 bis 9, dadurch gekennzeichnet, dass es zusätzlich Netzmittel, Emulgatoren und/oder Verdicker enthält.

11. Verfahren zum oxidativen Färben von Haaren, dadurch gekennzeichnet, dass man ein, eine Kombination von in der Haarfärbung bekannten Entwicklersubstanzen mit mindestens einem substituierten Phenol der allgemeinen Formel

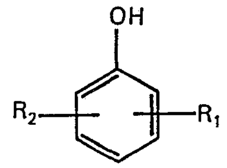

worin $R_1$ einen Hydroxylalkylrest mit 1 bis 4 Kohlenstoffatomen bedeutet und $R_2$ ein Wasserstoffatom oder einen Alkylrest mit 1 bis 4 Kohlenstoffatomen darstellt, auch in Form des Phenolates mit organischen oder anorganischen Basen, als Kupplersubstanz sowie gegebenenfalls zusätzlich bekannte Kupplersubstanzen enthaltendes Haarfärbemittel gemäss den Ansprüchen 1 bis 10 nach Zugabe eines Oxidationsmittels, insbesondere Hydrogenperoxid, auf die Haare aufbringt, etwa 10 bis 45 Minuten lang bei einer Temperatur von 15 bis 50°C einwirken lässt, anschliessend die Haare spült, gegebenenfalls wäscht und nachspült, und sodann trocknet.

## Claims

1. Composition for the oxidative colouring of hair, characterised in that it contains a combination of developer substances known in the hair colouring art with 0.01 to 3.0 weight percent of at least one substituted phenol of the general formula

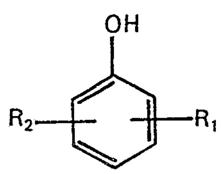

in which $R_1$ means a hydroxyalkyl residue with 1 to 4 carbon atoms and $R_2$ represents a hydrogen atom or an alkyl residue with 1 to 4 carbon atoms, which may be in the form of a phenolate with organic or inorganic bases, as coupler substance, as well as optionally additonal known coupler substances, so that the total amount of the developer substance – coupler substance combination present amounts to 0.1 to 5.0 weight percent.

2. Composition according to Claim 1, characterised in that the substituted phenol is selected from 3-(β-hydroxyethyl)-phenol 3-(γ-hydroxypropyl)-phenol, 3-(β-hydroxyethyl)-6-methyl-phenol, 2-(β-hydroxyethyl)-5-methyl-phenol, 2-hydroxymethyl-5-methyl-phenol and 2-hydroxymethyl-6-methyl-phenol.

3. Composition according to Claim 1 or 2, characterised in that it contains the coupler substance according to the invention in a concentration of 0.1 to 2.0 weight percent.

4. Composition according to Claim 1 to 3, characterised in that it contains at least one of the developer substances 1,4-diaminobenzene, 2,5-diaminotoluene, 2,5-diaminoanisole, 2-(β-hydroxyethyl)-1,4-diaminobenzene, 2,5-diaminobenzyl alocohol, p-aminophenol and 3-methyl-4-aminophenol.

5. Compositon according to Claim 1 to 4, characterised in that it contains at least one of the coupler substances α-naphthol, 3,4-diaminobenzoic acid, resorcinol, 4-chlorresorcinol, 2-methylresorcinol, m-aminophenol, 3-amino-6-methylphenol, 4-hydroxy-1,2-methylene dioxybenzene and 4-amino-1,2-methylene dioxybenzene.

6. Compostion according to Claim 1 to 5, characterised in that the total amount of the developer substance-coupler substance combination present amounts to 0.5 to 3.0 weight percent.

7. Composition according to Claim 1 to 6, characterised in that it additionally contains as colouring components 6-amino-3-methyl-phenol, 6-amino-2-methylphenol and 6-amino-3-ethoxyphenol.

8. Composition according to Claim 1 to 7, characterised in that it additionally contains at least one of the direct dyes Diamond Fuchsin (C.I. 42 510), Leather Ruby HF (C.I. 42 520), 2-nitro-1,4-diaminobenzene, 2-amino-4-nitrophenol, Acid Brown (4 C.I. 14 805), Acid Blue 135 (C.I. 13 385), Disperse Red 15 (C.I. 60 710), Disperse Violet 1 (C.I. 61 100), 1,4,5,8-tetraaminoanthraquinone and 1,4-diaminoanthraquinone.

9. Composition according to Claim 1 to 8, characterised in that it additionally contains antioxidants, preferable ascorbic acid or sodium sulphite.

10. Compositin according to Claim 1 to 9, characterised in that it additionally contains wetting agents, emulsifiers, and/or thickeners.

11. Process for the oxidative colouring of hair, characterised in that, after application of an oxidative composition, especially hydrogen peroxide, there is applied to the hair a hair colouring composition according to Claims 1 to 10 containing a combination of developer substances known in the hair colouring art with, as coupler substance, and optionally with the addition of known coupler substances, at least one substituted phenol of the general formula:

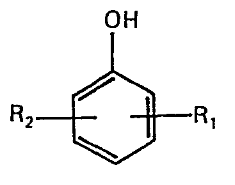

in which $R_1$ means a hydroxyalkyl residue with 1 to 4 carbon atoms and $R_2$ represents a hydrogen atom or an alkyl residue with 1 to 4 carbon atoms which may also be in the form of a phenolate with organic or inorganic bases, this is allowed to act for about 10 to 45 minutes at a temperature of 15 to 50°C, the hair is then rinsed, optionally washed and further rinsed, and then dried.

**Revendications**

1. Composition de coloration des cheveux par oxydation, caractérisée en ce qu'elle renferme une combinaison de substances de développement connues dans la coloration des cheveux comportant 0,01 à 3,0% en poids d'au moins un phénol substitué répondant à la formule générale

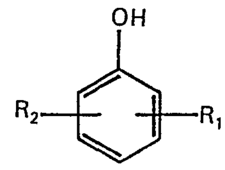

dans laquelle $R_1$ représente un reste hydroxyalcoyle comportant 1 à 4 atomes de carbone et $R_2$ un atome d'hydrogène ou un reste alcoyle comportant 1 à 4 atomes de carbone, pouvant également se présenter sous la forme du phénolate avec des bases organiques ou inorganiques, comme substance de copulation, ainsi éventuellement que des substances de copulation connues supplémentaires, de telle façon que la proportion totale de la combinaison de substances de développement et de substances de copulation qu'il renferme soit de 0,1 à 5,0% en poids.

2. Composition selon la revendication 1, caractérisée en ce que le phénol substitué est choisi parmi le 3-(β-hydroxyéthyl)-phénol, le 3-(γ-hydroxypropyl)-phénol, le 3-(β-hydroxyéthyl)-6-méthyl-phénol, le 2-(β-hydroxyéthyl)-5-méthylphénol, le 2-hydroxyméthyl-5-méthyl-phénol et le 2-hydroxyméthyl-6-méthyl-phénol.

3. Composition selon les revendications 1 et 2, caractérisée en ce qu'elle renferme les substances de copulation selon l'invention dans une concentration de 0,1 à 2,0% en poids.

4. Composition selon les revendications 1 à 3, caractérisée en ce qu'elle renferme au moins l'une des substances de développement suivantes: le 1,4-diaminobenzène, le 2,5-diaminotoluène, le 2,5-diamino-anisole, le 2-(β-hydroxyéthyl)-1,4-diaminobenzène, l'alcool 2,5-diaminobenzylique, le p-aminophénol et le 3-méthyl-4-aminophénol.

5. Composition selon les revendications 1 à 4, caractérisée en ce qu'elle renferme l'une au moins des substances de copulation suivantes: le α-naphthol, l'acide 3,4-diaminobenzoique, la résorcine, la 2-méthylrésorcine, le m-aminophénol, le 3-amino-6-méthylphénol, le 4-hydro xy-1,4-méthylène-dioxybenzène et le 4-amino-1,2-méthylène-dioxybenzène.

6. Composition selon les revendicvations 1 à 5, caractérisée en ce que la proportion totale de la combinaison des substances de développement et des substances de copulation qu'elle renferme est de 0,5 à 3,0% en poids.

7. Composition selon les revendications 1 à 6, caractérisée en ce qu'elle renferme en plus comme constituants de coloration le 6-amino-3-méthylphénol, le 6-amino-2-méthylphénol et le 6-amino-3-éthoxyphénol.

8. Composition selon les revendications 1 à 7, caractérisée en ce qu'elle renferme en plus au moins l'un des colorants à absorption directe suivants: Diamond Fuchsin (I.C. 42 510), Leather Ruby HF (I.C. 42 520), le 2-nitro-1,4-diaminobenzène, le 2-amino-4-nitrophénol, Acid Brown 4 (I.C. 14 805), Acid Blue 135 (I.C. 13 385), Disperse Red 15 (I.C. 60 710), Disperse Violet 1 (I.C. 61 100), la 1,4,5,8-tétra-amino-anthraquinone et la 1,4-diamino-anthraquinone.

9. Composition selon les revendications 1 à 8, caractérisée en ce qu'elle renferme en plus des anti-oxydants, de préférence de l'acide ascorbique ou du sulfite de sodium.

10. Composition selon les revendications 1 à 9, caractérisée en ce qu'elle renferme en plus des agents mouillants, des agents émulsionnants et/ou des épaississants.

11. Procédé de coloration par oxydation des cheveux, caractérisée en ce qu'on applique aux cheveux une composition de coloration des cheveux renfermant une combinaison de substances de développement connues dans la coloration des cheveux comportant au moins un phénol substitué répondant à la formule générale

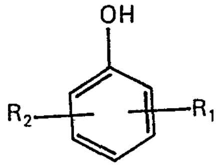

dans laquelle $R_1$ représente un reste hydroxyalcoyle comportant 1 à 4 atomes de carbone et $R_2$ un atome d'hydrogène ou un reste alcoyle comportant 1 à 4 atomes de carbone, pouvant également se présenter sous la forme du phénolate avec des bases organiques ou inorganiques comme substances de copulation, ainsi qu'éventuellement des substances de copulation connues supplémentaires, selon les revendications 1 à 10, après avoir ajouté un oxydant, notamment de l'eau oxygénée, on laisse l'action se poursuivre environ 10 à 45 minutes à une température de 15 à 50°C, puis on rince les cheveux, on les lave éventuellement en leur faisant subir un rinçage subséquent, puis on les sèche.